# EUROPÄISCHE PATENTSCHRIFT

(11) **EP 0 783 880 B1**
(45) Veröffentlichungstag und Bekanntmachung des Hinweises auf die Patenterteilung: **18.08.2004**
(21) Anmeldenummer: 96250293.6
(22) Anmeldetag: 18.12.1996
(51) Int. Cl.: A61K 6/083

(54) **Lichthärtendes Kompositmaterial**
Light curable composite material
Matériau composite durcissable par la lumière

(30) Priorität: 12.01.1996 DE 19601924
(43) Veröffentlichungstag der Anmeldung: 16.07.1997
(73) Patentinhaber: Ivoclar Vivadent AG, 9494 Schaan (LI)
(72) Erfinder: Moszner, Norbert, Prof. Dr., 9494 Schaan (LI); Rheinberger, Volker, Dr., 9490 Vaduz (LI)
(74) Vertreter: UEXKÜLL & STOLBERG

(56) Entgegenhaltungen:
- EP-A- 0 157 738
- EP-A- 0 659 749
- DE-A- 2 724 260
- DE-A- 3 821 091
- US-A- 4 839 401
- DATABASE WPI Section Ch, Week 198415 Derwent Publications Ltd., London, GB; Class A41, AN 1984-094116 XP002133896 & SU 1 027 150 A (SARAT UNIV MECHANIC), 7. Juli 1983 (1983-07-07)
- DATABASE WPI Section Ch, Week 199634 Derwent Publications Ltd., London, GB; Class A96, AN 1996-336899 XP002133897 & JP 08 155024 A (NIPPON ELECTRIC GLASS CO), 18. Juni 1996 (1996-06-18) & JP 08 155024 A (NIPPON ELECTRIC GLASS CO) 18. Juni 1996 (1996-06-18)

## Beschreibung

Die Erfindung betrifft ein lichthärtendes Kompositmaterial, welches infolge eines Gehaltes an speziellen Polymerisationsinhibitoren eine reduzierte Lichtempfindlichkeit sowie eine verbesserte Stabilität gegenüber der vorzeitigen Polymerisation beim Entlüften, z.B. durch Anlegen von Unterdruck, zeigt. Weiter betrifft die Erfindung ebenfalls die Verwendung des Kompositmaterials sowie geformte Dentalprodukte.

Lichthärtende Kompositmaterialien sind Zusammensetzungen, die neben lichthärtenden Monomeren Füllstoffe sowie ggf. weitere Zusatzstoffe enthalten. Im Dentalbereich eingesetzte lichthärtende Kompositmaterialien sind Einkomponentensysteme, die sich durch eine schnelle Aushärtung infolge Polymerisation, eine gute Lagerstabilität, eine geringe Verfärbung durch das eingesetzte Polymerisationsinitiatorsystem sowie eine sehr geringe Porosität nach der Aushärtung auszeichnen. Im Gegensatz zu den kalthärtenden Kompositmaterialien werden die lichthärtenden Komposite im Dentalbereich vor allem als Füllungskomposite oder Zemente in breitem Umfang eingesetzt (vgl. P. Riethe, Kariesprophylaxe und konservierende Therapie in "Farbatlanten der Zahnmedizin" Band 6, Seite 190, G. Thieme Verlag, Stuttgart-New York 1994). Zur Stabilisierung von lichthärtbaren dentalen Füllungskompositen, d.h. zur Verhinderung der vorzeitigen Polymerisation dieser Materialien, werden ihnen sogenannte Stabilisatoren, insbesondere substituierte Phenole, z.B. 2,6-Di-tert.-butyl-4-methylphenol (BHT) oder Hydrochinonmonomethylether (MEHQ), zugesetzt (vgl. J. Viohl et al., Die Chemie zahnärztlicher Füllungskunststoffe, C. Hanser Verlag, München-Wien 1986, Seite 27).

Ein Nachteil der bekannten lichthärtenden dentalen Kompositmaterialien und insbesondere der sogenannten hybriden Kompositmaterialien besteht jedoch darin, daß es bei ihrer Handhabung, z.B. in Form von üblicherweise verwendeten Pasten, infolge intensiver Beleuchtung oder durch Sonnenlicht zu einer unerwünschten vorzeitigen Aushärtung kommen kann (vgl. P. Dionysopoulos, D.C. Watts, J. Oral Rehabil. 17 (1990) 9). Dementsprechend wird in der internationalen Norm für dentale Füllungsmaterialien auf Kompositbasis (ISO 4049: 1988) gefordert, daß diese bei einer Beleuchtungsstärke von ca. 10000 lx mindestens 60 Sekunden lang stabil sein sollen, d.h. nicht aushärten.

Die Lichtempfindlichkeit eines dentalen Kompositmaterials wird herkömmlicherweise durch geeignete Abstimmung der Zusammensetzung und Konzentration des eingesetzten Photoinitiators sowie durch eine gezielte Auswahl der verwendeten Füllstoffkomponenten eingestellt. Eine Verringerung der Lichtempfindlichkeit durch Erhöhung der Konzentration der eingesetzten Stabilisatoren führt in der Regel nicht zum Ziel. Das liegt wahrscheinlich daran, daß es sich bei den üblicherweise als Stabilisatoren eingesetzten phenolischen Verbindungen, wie MEHQ, um sogenannte aerobe Stabilisatoren handelt, die nur in Verbindung mit in dem Kompositmaterial gelöstem Sauerstoff wirksam sind und dabei das Wachstum der Polymerradikale über mit dem Sauerstoff gebildete Peroxyradikale inhibieren (vgl. J. J. Kurland, J. Polym. Sci., Polym. Chem. Ed. 18 (1980) 1139). Da aber die Sättigungskonzentration von Sauerstoff in den meisten der in den Kompositmaterialien verwendeten Monomeren im Bereich von weniger als 100 ppm liegt, führt eine deutlich darüber hinaus gehende Konzentration an herkömmlichem Stabilisator zu keiner wesentlichen Verringerung der Lichtempfindlichkeit (vgl. M.J. Fried, Farbe und Lack 100 (1994) 604).

Neben den mit einer zu hohen Lichtempfindlichkeit einhergehenden Problemen können dentale Füllungskomposite hinsichtlich ihrer mechanischen Eigenschaften auch durch Lufteinschlüsse und daraus resultierenden Porositäten nachteilig beeinflußt werden, die dann auftreten, wenn die Kompositmaterialien wie üblich in Form von Pasten verarbeitet werden. Zur Verbesserung der Materialeigenschaften der Kompositmaterialpasten werden diese daher nach ihrer Herstellung in der Regel im Vakuum entlüftet. Dieses Entlüften kann jedoch bereits zu einer unkontrollierten Polymerisation und demzufolge einer vorzeitigen Aushärtung und Beeinträchtigung der Eigenschaften der Kompositmaterialien führen. Die vorzeitige Aushärtung ist darauf zurückzuführen, daß durch das Entlüften die Sauerstoffkonzentration in den Kompositpasten verringert und damit auch die Wirksamkeit der üblicherweise verwendeten aeroben Stabilisatoren reduziert wird.

Neben den herkömmlichen aeroben Stabilisatoren sind weiterhin auch Substanzen bekannt, die die radikalische Vinylpolymerisation auch in Abwesenheit von Sauerstoff wirksam inhibieren können und deshalb auch als anaerobe Stabilisatoren bezeichnet werden. Ein solcher anaerober Stabilisator ist z.B. Phenothiazin, das die Polymerisation von Acrylsäure im Gegensatz zu MEHQ auch in Abwesenheit von Sauerstoff inhibieren kann (vgl. L.B. Levy, J. Polym. Sci., Part A, Polym. Chem. 30 (1992) 569). Schließlich sind auch sogenannte stabile organische Radikale in der Literatur beschrieben, die als Polymerisationsinhibitoren eingesetzt werden können. Beispiele für solche stabilen organischen Radikale sind das 2,2-Diphenyl-1-picrylhydrazyl (DPPH)-, das Galvinoxyl-, das Triphenylmethyl- und das 2,2,6,6-Tetramethylpiperidinyl-1-oxyl-Radikal (vgl. E.S. Gould, Mechanismus und Struktur in der organischen Chemie, Verlag Chemie, Weinheim, 1962, S. 816).

Aus der US 4,839,401 sind lichthärtbare Fissurenversiegler bekannt, die 2,2-Propan-bis[3-(4-phenoxy)-1,2-dihydroxypropan-1-methacrylat] und Triethylenglykoldimethacrylat in einem Gewichtsverhältnis von 55:45, einen Polymerisationsinitiator, Titandioxid und Diethylaminoethylmethacrylat (DEA-EMA) als Polymerisationsbeschleuniger enthalten. Die Fissurenversiegler können bis zu 50 % Füllstoff enthalten. Das DEA-EMA enthält vorzugsweise 50 bis 100 ppm Phenothiazin.

Da sowohl die anaeroben Stabilisatoren als auch die stabilen organischen Radikale sehr wirksame Radikalfänger sind, ist damit zu rechnen, daß deren Einarbeitung in Kompositmaterialien eine nur unvollständige Aushärtung bewirkt, was sehr nachteilig wäre, da damit eine deutliche Verschlechterung der mechanischen Eigenschaften des ausgehärteten Materials einhergehen würde.

Der Erfindung liegt demgemäß die Aufgabe zugrunde, ein lichthärtendes Kompositmaterial zur Verfügung zu stellen, welches beim Entlüften nicht vorzeitig polymerisiert und dessen Lichtempfindlichkeit in der Weise eingestellt ist, daß eine durch Tageslicht induzierte Polymerisation inhibiert wird, jedoch eine durch Licht im Wellenlängenbereich von 400-550 nm induzierte Polymerisation nicht behindert und somit eine wesentliche Verschlechterung der mechanischen Eigenschaften des ausgehärteten Kompositmaterials vermieden wird.

Diese Aufgabe wird durch das lichthärtende Kompositmaterial nach den Ansprüchen 1 bis 9 gelöst. Die Erfindung betrifft ebenfalls die Verwendung des Kompositmaterials nach den Ansprüchen 10 und 11 sowie das geformte Dentalprodukt nach den Ansprüchen 12 und 13.

Das erfindungsgemäße lichthärtende Kompositmaterial ist dadurch gekennzeichnet, daß es (a) mindestens ein lichthärtendes Monomer, (b) mindestens einen Füllstoff und (c) mindestens einen anaeroben Stabilisator und/oder stabile organische Radikale enthält und mit Licht im Wellenlängenbereich von 400 bis 550 nm härtbar ist.

Anaerobe Stabilisatoren sind solche Verbindungen, die die radikalische Vinylpolymerisation auch in Abwesenheit von Sauerstoff inhibieren können. Diese anaeroben Stabilisatoren sind in einer Menge von 0,001 bis 1,0, insbesondere 0,001 bis 0,50 und besonders bevorzugt 0,001 bis 0,20 Gew.-% in dem erfindungsgemäßen Kompositmaterial enthalten.

Besonders vorteilhafte anaerobe Stabilisatoren sind Phenothiazin und/oder die der folgenden Formel I entsprechenden Derivate von Phenothiazin 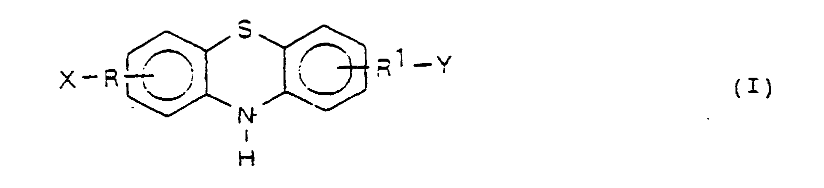 wobei R, R¹, X, Y, R², R³, R⁴, R⁵, R⁶ und R⁷ unabhängig voneinander die folgenden Bedeutungen haben:
- R und R¹ =: C₁- bis C₅-Alkylen oder -Oxyalkylen oder C₆-bis C₁₂-Arylen
- X und Y =: H, Halogen, NO₂, NH₂, NR²R³, OH, OR⁴, CN, CHO, CO-R⁵, COOH, CO-NH₂, CO-OR⁶, CH₂=CH-, CH₂=CH-CO-, CH₂=C(CH₃)-CO-, SH oder S-R⁷
- R² bis R⁷=: Alkyl, Alkenyl, Aryl, Alkylaryl oder Arylalkyl mit jeweils 1 bis 12 C-Atomen.

Anstelle von oder zusätzlich zu den anaeroben Stabilisatoren enthält das erfindungsgemäße Kompositmaterial sogenannte stabile organische Radikale. Unter stabilen organischen Radikalen sind solche organischen Radikale zu verstehen, die bei Raumtemperatur in festem Zustand oder in Lösung eine Halbwertszeit von mindestens 30 Tagen, insbesondere von mindestens 12 Monaten, aufweisen. Die Radikalstabilität wird dabei bekanntlich (vgl. H.A. Staab, Einführung in die theoretische organische Chemie, Verlag Chemie, Weinheim 1966, S. 442 ff.) vor allem durch die Mesomeriestabilisierung der Radikale oder die Destabilisierung der entsprechenden dimeren Kombinationsprodukte durch sterische Effekte hervorgerufen. Es sind u.a. stabile Kohlenstoff-Radikale, wie Tribiphenylmethyl-, stabile Sauerstoff-Radikale, wie Galvinoxyl-, oder stabile Stickstoff-Radikale, wie DPPH-Radikale, bekannt.

Typischerweise sind die stabilen organischen Radikale in einer Menge von 0,001 bis 1,0, insbesondere 0,001 bis 0,50 und besonders bevorzugt 0,001 bis 0,20 Gew.-% in dem erfindungsgemäßen Kompositmaterial enthalten.

Dabei werden als stabile organische Radikale vorzugsweise 2,2-Diphenyl-1-picrylhydrazyl- (DPPH), Galvinoxyl- und/oder Triphenylmethylradikale eingesetzt. Besonders bevorzugt sind in den erfindungsgemäßen Materialien jedoch 2,2,6,6-Tetramethylpiperidinyl-1-oxylradikale (TEMPO) oder Derivate von TEMPO der nachstehenden Formeln (II) und (III) enthalten. 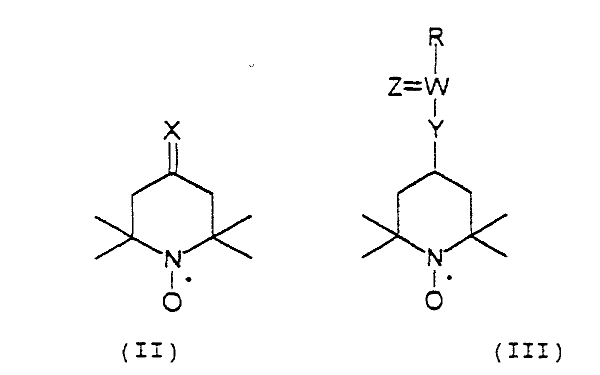 wobei X, Y, Z, W und R unabhängig voneinander die folgenden Bedeutungen haben:
- X =: O oder S
- Y und Z =: O, S oder entfällt
- W =: C oder entfällt
- R =: H oder Alkyl, Alkenyl, Aryl, Alkylaryl oder Arylalkyl mit jeweils 1 bis 12 C-Atomen.

Die Einstellung der bei den Kompositmaterialien jeweils gewünschten Lichtempfindlichkeit und Stabilität beim Entlüften, auch als Vakuumstabilität bezeichnet, erfolgt durch geeignete Wahl der Menge und Art des anaeroben Stabilisators und/oder der stabilen organischen Radikalen. Es hat sich insbesondere gezeigt, daß selbst sehr kleine Mengen an anaerobem Stabilisator und/oder stabilen organischen Radikalen die unerwünschte durch Tageslicht induzierte Polymerisation der Kompositmaterialien wirksam verhindern, ohne daß es gleichzeitig zu einer Behinderung einer mittels Blaulicht (Wellenlänge 400 bis 550 nm) ausgelösten Polymerisation kommt. Letzteres ist vermutlich auch der Grund dafür, daß die Verwendung der erfindungsgemäß vorgesehenen Polymerisationsinhibitoren zu keiner wesentlichen Verschlechterung der mechanischen Eigenschaften beim ausgehärteten Material führt. So zeigt das ausgehärtete Material sowohl bei der Biegefestigkeit als auch beim Biege-E-Modul keine wesentlichen Verringerungen im Vergleich zu einem Material ohne Gehalt an anaerobem Stabilisator und/oder stabilen organischen Radikalen.

Zur Gewährleistung der normalen Lagerstabilität enthält das erfindungsgemäße Kompositmaterial vorzugsweise auch herkömmliche Polymerisationsinhibitoren. Beispiele für solche herkömmlichen Polymerisationsinhibitoren sind 2,6-Di-tert.-butyl-4-methylphenol (BHT) und Hydrochinonmonomethylether (MEHQ). Typischerweise sind die herkömmlichen Polymerisationsinhibitoren in einer Menge von bis zu 1,0 Gew.-% in dem Kompositmaterial enthalten.

Es zeigte sich, daß die Verwendung von herkömmlichen Polymerisationsinhibitoren alleine, selbst in größeren Mengen, nicht zu einer wesentlichen Verringerung der Lichtempfindlichkeit oder einer Verbesserung der Vakuumstabilität von Kompositpasten führten. Dies konnte nur mittels des anaeroben Stabilisators und/oder der stabilen organischen Radikale erreicht werden.

Das erfindungsgemäße Kompositmaterial enthält außerdem mindestens ein lichthärtendes Monomer. Vorzugsweise finden hierfür ethylenisch-ungesättigte Monomere und insbesondere monofunktionelle oder polyfunktionelle Acrylate und/oder Methacrylate Verwendung. Bevorzugte Beispiele für diese sind Methyl(meth)acrylat, Isobutyl(meth)acrylat, Cyclohexyl(meth)acrylat, Tetraethylenglykoldi(meth)acrylat, Diethylenglykoldi(meth)acrylat, Ethylenglykoldi(meth)acrylat, Polyethylenglykoldi(meth)acrylat, Butandioldi(meth)acrylat, Hexandioldi(meth)acrylat, Decandioldi(meth)acrylat, Dodecandioldi(meth)acrylat, Bisphenol-A-di(meth)acrylat, Trimethylolpropantri(meth)acrylat, sowie die Produkte der Reaktion von Isocyanaten, insbesondere Di- und/oder Triisocyanaten, mit OH-gruppenhaltigen Methacrylaten. Beispiele für die zuletzt erwähnten Produkte sind die durch Umsetzung von 1 Mol Hexamethylendiisocyanat mit 2 Mol 2-Hydroxyethylenmethacrylat und von 1 Mol Tri-(6-isocyanatohexyl)biuret mit 3 Mol 2-Hydroxyethylmethacrylat erhaltenen Produkte. Besonders bevorzugt sind Triethylenglykoldi(meth)acrylat, 2,2-Bis-4-(3-Methacryloxy-2-hydroxypropoxy)-phenylpropan (Bis-GMA) und die durch Reaktion von 1 Mol 2,2,4-Trimethylhexamethylendiisocyanat mit 2 Mol 2-Hydroxyethylmethacrylat erhaltenen Produkte.

Bevorzugt beträgt die Gesamtmenge an lichthärtenden Monomeren in dem erfindungsgemäßen Kompositmaterial 7 bis 80, besonders bevorzugt 14 bis 50 Gew.-%.

Weiter enthält das erfindungsgemäße Kompositmaterial mindestens einen Füllstoff. Dabei sind besonders geeignete Füllstoffe anorganische Füllstoffe und insbesondere Quarz- oder Glaskeramik-Pulver, Aluminiumoxide, Mischoxide, wie SiO₂-ZrO₂-Mischoxid, oder röntgenopake Füllstoffe, wie Ytterbiumtrifluorid. Besonders bevorzugte Füllstoffe sind Glaspulver, z.B. Bariumglas-, Bariumsilikatglas-, Li- oder Al-Silikatglas-Pulver und feinteilige Kieselsäuren, wie pyrogene oder gefällte Kieselsäuren.

Die Füllstoffe werden vorzugsweise in einer Menge von 10 bis 90, insbesondere 48 bis 85 Gew.-% in dem erfindungsgemäßen Kompositmaterial eingesetzt.

Ferner enthält das Kompositmaterial vorzugsweise auch noch Initiatoren für die Photopolymerisation, wie z.B. Benzophenon, Benzoin oder Derivate von diesen. Bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon verwendet. Typischerweise werden die Photoinitiatoren in Kombination mit einem Amin als Aktivator eingesetzt, wobei z.B. N,N-Dimethyl-p-toluidin, N,N-Dihydroxyethyl-p-toluidin und insbesondere N-(2-Cyanoethyl)-N-methylanilin in Frage kommen.

Schließlich kann das erfindungsgemäße Kompositmaterial auch noch übliche Zusätze und vor allem bei dentalen Kompositmaterialien übliche Zusätze, wie Farbstoffe, Pigmente, Weichmacher, Gleitmittel, Rheologiemodifikatoren oder UV-Stabilisatoren enthalten.

Zur Herstellung des erfindungsgemäßen lichthärtenden Kompositmaterials werden die zur Herstellung von dentalen Kompositmaterialien üblichen Verfahren eingesetzt, wobei die erfindungsgemäß erforderlichen Stabilisatoren, nämlich der anaerobe Stabilisator und/oder die stabilen organischen Radikale, zu einem beliebigen Zeitpunkt während des Herstellungsverfahrens z.B. durch einfaches Zumischen in das Kompositmaterial eingearbeitet werden.

Das erfindungsgemäße Kompositmaterial zeichnet sich insbesondere dadurch aus, daß seine Lichtempfindlichkeit durch Gehalt an geringen Mengen an anaerobem Stabilisator und/oder stabilen organischen Radikalen so eingestellt ist, daß es weder beim Entlüften, z.B. durch Anlegen eines Vakuums, noch bei Belichtung mittels Tageslicht vorzeitig polymerisiert. Dabei kann sowohl die Vakuumstabilität als auch die Lichtempfindlichkeit einfach durch die Art und Menge an anaerobem Stabilisator und/oder stabilen organischen Radikalen gesteuert werden.

Die vorstehenden Eigenschaften machen das erfindungsgemäße Kompositmaterial insbesondere als dentales Füllungsmaterial geeignet. Es ist jedoch auch möglich, es im prothetischen Bereich, z.B. als Material zur Herstellung von Kronen oder Brücken, einzusetzen. Dabei erweist sich die verringerte Empfindlichkeit gegenüber Tageslicht als besonders vorteilhaft, da der Zahntechniker auch bei normalem diffusen Tageslicht die Modellierung von z.B. einer Kronen- oder Brückenkonstruktion vornehmen kann, ohne daß es dabei zu einer vorzeitigen Polymerisation kommen würde.

Die Erfindung betrifft ebenfalls die Verwendung des Kompositmaterials als Dentalmaterial und insbesondere die Verwendung des anaeroben Stabilisators und/oder der stabilen organischen Radikale zur Verringerung der Lichtempfindlichkeit des Dentalmaterials und zur Inhibierung der vorzeitigen Polymerisation des Dentalmaterials beim Entlüften.

Schließlich betrifft die Erfindung ebenfalls ein aus dem erfindungsgemäßen Kompositmaterial geformtes Dentalprodukt, insbesondere eine Füllung, eine Krone oder eine Brücke, welches das Kompositmaterial in zumindest teilweise gehärteter Form enthält. Die Verformung des Kompositmaterials erfolgt dabei in an sich bekannter Weise.

Im folgenden wird die Erfindung anhand von Beispielen näher erläutert.

### Beispiele

### Beispiel 1

Zunächst wurde aus den folgenden Substanzen eine Monomermischung hergestellt:
- Momomere:: - Bisphenol-A-glycidylmethacrylat (Bis-GMA)
- Urethandimethacrylat (UDMA), hergestellt aus 1 Mol 2,2,4-Trimethylhexamethylendiisocyanat und 2 Mol 2-Hydroxyethylmethacrylat
- Triethylenglykoldimethacrylat (TEGDMA)
- Photoinitiator:: - Kombination von Campherchinon (CC) und N-(2-Cyanoethyl)-N-methylanilin (CEMA)
- Inhibitor:: - Hydrochinonmonomethylether (MEHQ)

Die Monomermischung hatte die nachstehende Zusammensetzung:

| Komponente | Gew.-% |
|---|---|
| Bis-GMA | 41,77 |
| UDMA | 37,42 |
| TEGDMA | 20,00 |
| MEHQ | 0,01 |
| CC | 0,30 |
| CEMA | 0,50 |

Diese Monomermischung wurde dann mit Füllstoffen versetzt, um ein Kompositmaterial folgender Zusammensetzung herzustellen:

| Gew.-% | Komponente |
|---|---|
| 26,0 | Monomermischung |
| 44,0 | silanisiertes Bariumaluminiumsilikatglas (Glasfüller, Schott-Glaswerke) |
| 14,5 | Ytterbiumtrifluorid (Rhone-Poulenc) |
| 14,5 | silanisiertes sphärisches SiO₂-ZrO₂-Mischoxid (Sphärosil, Tokyamo Soda) |
| 1,0 | sitanisierte pyrogene Kieselsäure (Ox-50, Degussa) |

Zu diesem Kompositmaterial wurden dann geringe Mengen Phenothiazin als anaerober Stabilisator oder 2,2,6,6-Tetramethylpiperidinyl-1-oxyl-Radikale (TEMPO) oder 2,2-Diphenyl-1-picrylhydrazyl-Radikale (DPPH) als stabile organische Radikale zugegeben. Die angegebenen Mengen beziehen sich dabei auf die eingesetzte Menge an Kompositmaterial ohne Phenothiazin, TEMPO und DPPH. Bei Proben des erhaltenen Kompositmaterials mit zugesetztem Phenothiazin. oder TEMPO oder DPPH und zum Vergleich solchen ohne diese Zusätze wurden dann nach ISO 4049 die Lichtempfindlichkeit, die Biegefestigkeit und der Biege-E-Modul bestimmt. Die gemessenen Werte sind nachstehend aufgelistet.

| Zusatz (Gew.-%) | Lichtempfindlichkeit (s) | Biefestigkeit (MPa) | Biege-E-Modul (GPa) |
|---|---|---|---|
| ohne | 70 | 114 | 9,5 |

| Phenothiazin | | | |
|---|---|---|---|
| 0,03 | 95 | 111 | 9,5 |
| 0,05 | 105 | 121 | 10,9 |
| 0,10 | 130 | 110 | 9,5 |

| TEMPO | | | |
|---|---|---|---|
| 0,0025 | 105 | 117 | 9,5 |
| 0,005 | 150 | 121 | 9,5 |
| 0,01 | 240 | 124 | 9,4 |

| DPPH | | | |
|---|---|---|---|
| 0,10 | 145 | 124 | 11,6 |

Die Ergebnisse zeigen, daß durch Zugabe von Phenothiazin oder TEMPO oder DPPH die Lichtempfindlichkeit der Komposite deutlich verringert werden kann, ohne daß dies zu einer Verschlechterung der mechanischen Eigenschaften der Materialien führen würde.

Da in allen Fällen mit Licht der Wellenlänge 400-550 nm unter gleichen Bedingungen gehärtet wurde, belegen die obigen Werte für die Biegefestigkeit und dem Biege-E-Modul ebenfalls, daß bei den erfindungsgemäßen Kompositmaterialien trotz des Gehalts an anaerobem Stabilisator oder stabilen organischen Radikalen eine Behinderung der Lichthärtung nicht auftritt.

### Beispiel 2

Analog Beispiel 1 wurde ein Kompositmaterial folgender Zusammensetzung hergestellt:

| Gew.-% | Komponente |
|---|---|
| 19,0 | Monomermischung gemäß Beispiel 1 |
| 51,0 | silanisiertes Bariumaluminiumsilikatglas (Glasfüller) |
| 14,5 | Ytterbiumtrifluorid |
| 14,5 | silanisiertes sphärisches SiO₂-ZrO₂-Mischoxid (Sphärosil) |
| 1,0 | silanisierte pyrogene Kieselsäure (Ox-50) |

Die erhaltene Paste wurde im Kneter bei < 50 mbar entlüftet. Dabei begann die Paste, nach ca. 5 min. zu polymerisieren.

Eine Paste gleicher Zusammensetzung aber mit einem Zusatz vor 0,06 Gew.-% Phenothiazin (bezogen auf Menge der Paste vor Zugabe von Phenothiazin) war demgegenüber 20 min. lang stabil, was für ein vollständiges Entlüften ausreicht.

Bei einer Paste gleicher Zusammensetzung aber mit einem Zusatz von 0,005 Gew.-% TEMPO anstelle von Phenothiazin (bezogen auf Menge der Paste vor Zugabe von TEMPO) waren selbst nach 1 keine Polymerisationserscheinungen erkennbar.

## Patentansprüche

1. Lichthärtendes Kompositmaterial, **dadurch gekennzeichnet, daß** es
(a) mindestens ein lichthärtendes Monomer,
(b) mindestens einen Füllstoff und
(c) mindestens einen anaeroben Stabilisator in einer Menge von 0,001 bis 1,0 Gew.-% und/oder stabile organische Radikale enthält
und mit Licht im Wellenlängenbereich von 400 bis 550 nm härtbar ist.

2. Kompositmaterial nach Anspruch 1, **dadurch gekennzeichnet, daß** es als anaeroben Stabilisator Phenothiazin und/oder ein Derivat davon der folgenden Formel I enthält 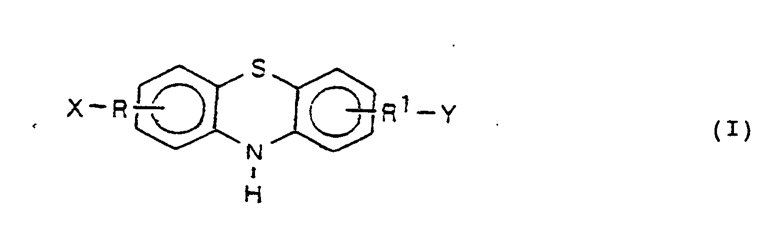 wobei R, R¹, X, Y, R², R³, R⁴, R⁵, R⁶ und R⁷ unabhängig voneinander die folgenden Bedeutungen haben:
R und R¹ = = C₁- bis C₅-Alkylen oder -Oxyalkylen oder C₆-bis C₁₂-Arylen
X und Y = = H, Halogen, NO₂, NH₂ , NR²R³, OH, OR⁴, CN, CHO, CO-R⁵, COOH, CO-NH₂, CO-OR⁶, CH₂=CH-, CH₂=CH-CO-, CH₂=C(CH₃)-CO-, SH oder S-R⁷
R² bis R⁷= Alkyl, Alkenyl, Aryl, Alkylaryl oder Arylalkyl mit jeweils 1 bis 12 C-Atomen

3. Kompositmaterial nach Anspruch 1 oder 2, **dadurch gekennzeichnet, daß** es insbesondere 0,001 bis 0,50 Gew.-% und besonders bevorzugt 0,001 bis 0,20 Gew.-% anaeroben Stabilisator enthält.

4. Kompositmaterial nach einem der Ansprüche 1 bis 3, **dadurch gekennzeichnet, daß** es als stabile organische Radikale 2,2-Diphenyl-1-picrylhydrazyl-, Galvinoxyl-, Triphenylmethyl-Radikale und/oder Radikale der Formeln II oder III, insbesondere 2,2,6,6-Tetramethylpiperidinyl-1-oxylradikale, enthält, 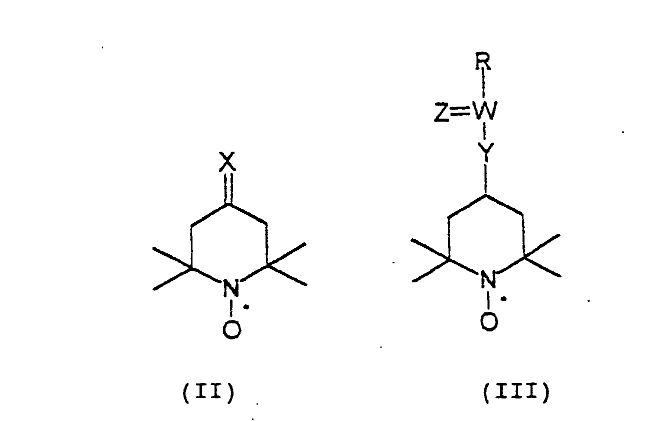 wobei X, Y, Z, W und R unabhängig voneinander die folgenden Bedeutungen haben:
X = O oder S
Y und Z = O, S oder entfällt
W = C oder entfällt
R = H oder Alkyl, Alkenyl, Aryl, Alkylaryl oder Arylalkyl mit jeweils 1 bis 12 C-Atomen.

5. Kompositmaterial nach einem der Ansprüche 1 bis 4, **dadurch gekennzeichnet, daß** es 0,001 bis 1,0 Gew.-%, insbesondere 0,001 bis 0,50 Gew.-% und besonders bevorzugt 0,001 bis 0,20 Gew.-% stabile organische Radikale enthält.

6. Kompositmaterial nach einem der Ansprüche 1 bis 5, **dadurch gekennzeichnet, daß** es herkömmliche Polymerisationsinhibitoren, insbesondere in einer Menge von bis zu 1,0 Gew.-%, enthält.

7. Kompositmaterial nach einem der Ansprüche 1 bis 6, **dadurch gekennzeichnet, daß** es 7 bis 80 Gew.% und insbesondere 14 bis 50 Gew.% lichthärtende Monomere (a) enthält.

8. Kompositmaterial nach einem der Ansprüche 1 bis 7, **dadurch gekennzeichnet, daß** es 10 bis 90 Gew.% und insbesondere 48 bis 85 Gew.% Füllstoffe (b) enthält.

9. Kompositmaterial nach einem der Ansprüche 1 bis 8, **dadurch gekennzeichnet, daß** es ein Kronen- oder Brückenmaterial, und insbesondere ein Füllungsmaterial ist.

10. Verwendung des Kompositmaterials gemäß einem der Ansprüche 1 bis 9 als Dentalmaterial.

11. Verwendung nach Anspruch 10, wobei der anaerobe Stabilisator und/oder die stabilen organischen Radikale zur Verringerung der Lichtempfindlichkeit des Dentalmaterials und zur Inhibierung der vorzeitigen Polymerisation des Dentalmaterials beim Entlüften eingesetzt werden.

12. Geformtes Dentalprodukt, **dadurch gekennzeichnet, daß** es das Kompositmaterial gemäß einem der Ansprüche 1 bis 9 in zumindest teilweise gehärteter Form enthält.

13. Dentalprodukt gemäß Anspruch 12, **dadurch gekennzeichnet, daß** es eine Füllung, ein Krone oder eine Brücke ist.

## Claims

1. Light curable composite material **characterised in that** it comprises
(a) at least one light curable monomer
(b) at least one filler and
(c) at least one anaerobic stabiliser in a quantity from 0.001 to 1.0 wt.% and/or a stable organic radical
and is curable with light in the wavelength region of 400 to 500 nm.

2. Composite material according to Claim 1 **characterised in that** the anaerobic stabiliser comprises phenothiazine and/or a derivative thereof of the following Formula I 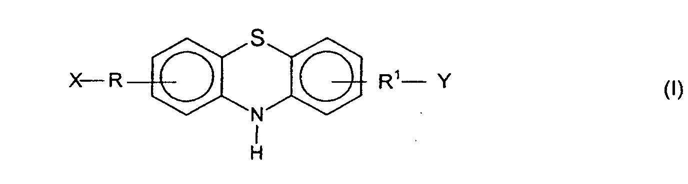 in which R, R¹, X, Y, R², R³, R⁴, R⁵, R⁶ and R⁷, independently of each other, have the following meanings:
R and R¹ = C₁ to C₅ alkylene or oxyalkylene or C₆ to C₁₂ arylene
X and Y = H, halogen, NO₂, NH₂, NR²R³, OH, OR⁴, CN, CHO, CO-R⁵, COOH, CO-NH₂, CO-OR⁶, CH₂=CH-, CH₂=CH-CO-, CH₂=C(CH₃)-CO-, SH or S-R⁷
R² to R⁷ = alkyl, alkenyl, aryl, alkylaryl or arylalkyl having in each case 1 to 12 C atoms.

3. Composite material according to Claim 1 or 2 **characterised in that** it preferably comprises 0.001 to 0.50 wt.% and particularly preferably 0.001 to 0.20 wt.% anaerobic stabiliser.

4. Composite material according to any one of Claims 1 to 3 **characterised in that** it comprises as the stable organic radical 2,2-diphenyl-1-picrylhydrazyl radicals, galvinoxyl radicals, triphenylmethyl radicals, and/or radicals of Formula II or III, particularly 2,2,6,6-tetramethylpiperidinyl-1-oxyl radicals 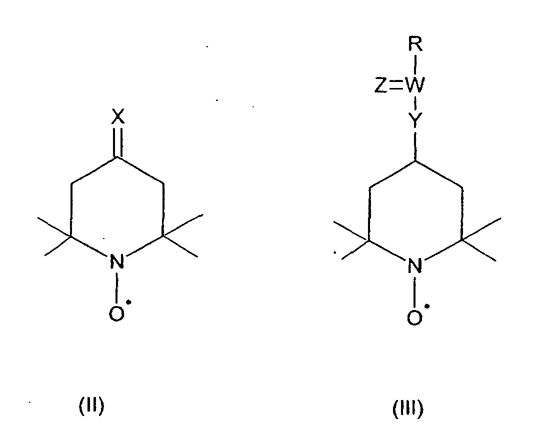 in which X, Y, Z, W and R, independently of each other, have the following meanings:
X = O or S
Y and Z = O, S or not present
W = C or not present
R = H or alkyl, alkenyl, aryl, alkylaryl or arylalkyl having in each case 1 to 12 C atoms.

5. Composite material according to any one of Claims 1 to 4 **characterised in that** it comprises 0.001 to 1.0 wt.%, particularly 0.001 to 0.50 wt.% and particularly preferably 0.001 to 0.20 wt.% stable organic radical.

6. Composite material according to one of Claims I to 5 **characterised in that** it comprises conventional polymerisation inhibitors particularly in a quantity up to 1.0 wt.%.

7. Composite material according to any one of Claims 1 to 6 **characterised in that** it comprises 7 to 80 wt.%, particularly 14 to 50 wt.% light curable monomer (a).

8. Composite material according to any one of Claims 1 to 7 **characterised in that** it comprises 10 to 90 wt.%, particularly 48 to 85 wt.% filler (b).

9. Composite material according to one of Claims 1 to 8 **characterised in that** it is a crown or bridge material and particularly a dental filling material.

10. Use of the composite material according to one of the Claims 1 to 9 as a dental material.

11. Use according to Claim 10, whereby the anaerobic stabiliser and/or the stable organic radical are employed to reduce the light sensitivity of the dental material and to inhibit premature polymerisation of the dental material during deaeration.

12. Moulded dental product, **characterised in that** it comprises the composite material according to one of the Claims 1 to 9, in at least a partially cured state.

13. Dental product according to Claim 12 **characterised in that** it is a filling, a crown or a bridge.

## Revendications

1. Matériau composite photodurcissable, **caractérisé en ce qu'**il contient
(a) au moins un monomère photodurcissable,
(b) au moins une matière de charge, et
(c) au moins un agent stabilisant anaérobie en une quantité de 0,001 à 1,0% en poids et/ou des radicaux organiques stables,
et **en ce qu'**il peut être durci par de la lumière dans la plage de longueur d'onde de 400 à 550 nm.

2. Matériau composite selon la revendication 1, **caractérisé en ce qu'**il contient comme agent stabilisant anaérobie de la phénothiazine et/ou un dérivé de celle-ci de la formule 1 : 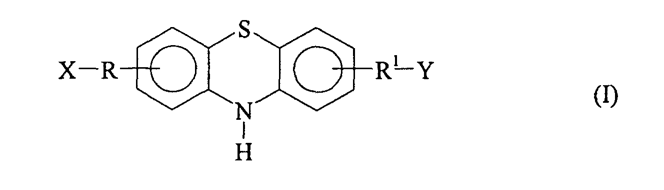 dans laquelle R, R¹, X, Y, R², R³, R⁴, R⁵, R⁶ et R⁷ ont indépendamment les uns des autres les significations suivantes :
R et R¹ = alkylène ou oxyalkylène en C₁ à C₅ ou arylène en C₆ à C₁₂,
X et Y = H, halogène, NO₂, NH₂, NR²R³, OH, OR⁴, CN, CHO, CO-R⁵, COOH, CO-NH₂, CO-OR⁶, CH₂=CH-, CH₂=CH-CO-, CH₂=C(CH₃)-CO-, SH ou S-R⁷,
R² à R⁷ = alkyle, alcényle, aryle, alkylaryle ou arylalkyle possédant chacun de 1 à 12 atomes de carbone.

3. Matériau composite selon la revendication 1 ou la revendication 2, **caractérisé en ce qu'**il contient en particulier de 0,001 à 0,50% en poids et de façon particulièrement préférée de 0,001 à 0,20% en poids d'agent stabilisant anaérobie.

4. Matériau composite selon l'une des revendications 1 à 3, **caractérisé en ce qu'**il contient comme radicaux organiques stables des radicaux 2,2-diphényl-1-picrylhydrazyle (DPPH), galvinoxyle, triphénylméthyle et/ou des radicaux des formules II et III, en particulier des radicaux 2,2,6,6-tétraméthylpipéridinyl-1-oxyle : 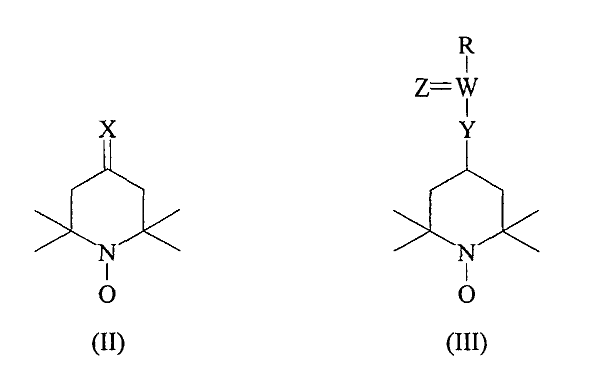 dans lesquelles X, Y, Z, W et R ont indépendamment les uns des autres les significations suivantes :
X = O ou S,
Y et Z = O, S ou absents,
W = C ou absent,
R = H ou alkyle, alcényle, aryle, alkylaryle ou arylalkyle possédant chacun de 1 à 12 atomes de carbone.

5. Matériau composite selon l'une des revendications 1 à 4, **caractérisé en ce qu'**il contient de 0,001 à 1,0% en poids, en particulier de 0,001 à 0,50% en poids et de façon particulièrement préférée de 0,001 à 0,20% en poids de radicaux organiques stables.

6. Matériau composite selon l'une des revendications 1 à 5, **caractérisé en ce qu'**il contient des inhibiteurs de polymérisation classique, notamment en une quantité allant jusqu'à 1,0% en poids.

7. Matériau composite selon l'une des revendications 1 à 6, **caractérisé en ce qu'**il contient de 7 à 80% en poids et en particulier de 14 à 50% en poids de monomères photodurcissables (a).

8. Matériau composite selon l'une des revendications 1 à 7, **caractérisé en ce qu'**il contient de 10 à 90% en poids et en particulier de 48 à 85% en poids de matières de charge (b).

9. Matériau composite selon l'une des revendications 1 à 8, **caractérisé en ce qu'**il s'agit d'un matériau pour des couronnes ou des bridges et en particulier d'un matériau d'obturation.

10. Utilisation du matériau composite selon l'une des revendications 1 à 9 à titre de matériau dentaire.

11. Utilisation selon la revendication 1, dans laquelle l'agent stabilisant anaérobie et/ou les radicaux organiques stables sont utilisés pour réduire la sensibilité à la lumière du matériau dentaire et pour inhiber la polymérisation prématurée du matériau dentaire lors du dégazage.

12. Produit dentaire formé, **caractérisé en ce qu'**il contient le matériau composite selon l'une des revendications 1 à 9 sous une forme durcie au moins en partie.

13. Produit dentaire selon la revendication 12, **caractérisé en ce qu'**il s'agit d'une obturation, d'une couronne ou d'un bridge.
